# EUROPEAN PATENT APPLICATION

(11) **EP 2 484 343 A1**
(43) Date of publication of application: **08.08.2012**
(21) Application number: 10770699.6
(22) Date of filing: 28.09.2010
(51) Int. Cl.: A61K 9/14, A61K 39/29, A61P 31/12

(54) **HEPATITIS B VIRUS ANTIGEN FORMULATION FOR CELL STIMULATION FOLLOWED BY THERAPEUTIC IMMUNIZATION**

(30) Priority: 29.09.2009 CU 20090164
(71) Applicant: Centro De Ingenieria Genetica Y Biotecnologia, Ciudad De La Habana 10600 (CU)
(72) Inventor: AGUILAR RUBIDO, Julio, La Habana 32200 (CU); LOBAINA MATO, Yadira, Ciudada de la Habana 11600 (CU); HERNÁNDEZ INGUANZO, Dunia, Ciudad de la Habana 11300 (CU); TRUJILLO PÉREZ, Heidy, Ciudad de la Habana 11500 (CU); FREYRE ALMEIDA, Freya, de los Milagros, Ciudad de la Habana 10400 (CU); MUZIO GONZÁLEZ, Verena Lucila, Ciudad de la Habana 10400 (CU); PENTÓN ARIAS, Eduardo, Ciudad de la Habana 12100 (CU); GONZALEZ BLANCO, Sonia, Ciudad de la Habana 11600 (CU); UBIETA GÓMEZ, Raimundo, Ciudad de la Habana 11600 (CU); GUILLÉN NIETO, Gerardo Enrique, Ciudad de la Habana 10400 (CU); HERRERA MARTINEZ, Luis Saturnino, Ciudad de la Habana 10600 (CU)
(74) Representative: Jansen, Cornelis Marinus
(86) International application number: PCT/CU2010/000003
(87) International publication number: WO 2011/038701

(57) **Abstract**

The present invention is related with the field of the therapeutic immunization, specifically with the employment of a new formulation of antigens of the Hepatitis B Virus (HBV) for the cellular stimulation. The formulation is formed by the surface antigens (HBsAg) precipitated in suspension and the nucleocapsid (HBcAg) of the HBV. The formulation contains these antigens like suspended particles of sizes less than 500 nm and higher than 500 nm, in a mixture where the proportion among the particles of the mentioned sizes is among 50% - 50% and 80% - 20%, respectively. The selection of a range of sizes of particles allows that the levels of stimulation of several cellular types are maximized. Additionally, a cellular stimulation method is described with this formulation, and the later passive immunization of patients with chronic Hepatitis B, based on the maximum stimulation *in vivo* or *in vitro* using heterologous or autologous cells (dendritic cells, B cells and macrophages). The cells stimulated with this formulation are transferred to patients chronically infected with the HBV.

## Description

### Technical field

The present invention is related with medicine, particularly with the therapeutic immunization by means of the transfer of cells that have been stimulated with a formulation of the surface (HBsAg) and nucleocapsid (HBcAg) antigens of the Hepatitis B Virus (HBV). The cellular stimulation is induced with a formulation that contains a mixture of these antigens, precipitated in suspension which proportions have been selected based on achieving the maximum cellular stimulation before the passive immunotherapeutic use.

### Background of the invention

The World Health Organization (WHO) considers that more than a third of the world's population has been infected by the Hepatitis B Virus (HBV). It is considered that between 5 and 10% of adults, and until 90% of infected newborns develop the infection in a persistent way, with 350 millions of people carrying the virus at world level. The sustained replication of the HBV during a long period of time leads to a progressive hepatic illness that evolves to cirrhosis or liver cancer in approximately 25% of the carriers. It is considered that annually more than a million deaths are related to the infection by this virus in their different evolution forms (Zuckerman JN. J MED VIROL 2006; 78: 169-177).

The treatment with alpha Interferon (IFN-α), or its variant conjugated to polyethylene glycol (PEG) and nucleosides or nucleotides like the Lamivudine, Adefovir-dipivoxil, Entecavir and Telbivudina, constitutes the state of the art in the treatment of the chronic hepatitis B (CHB). In a general way, these drugs have a poor effectiveness in terms of sustained elimination of the virus, and their use is associated to important secondary effects (Osborn MK, et to the one. J ANTIMICROB CHEMOTHER. 2006; 57: 1030-34).

The development of a vaccine with a high percentage of effectiveness for the prevention of the hepatitis B constitutes an important achievement of the preventive medicine at a world level. At present, more than 150 countries offer the anti-hepatitis B vaccine to children, adolescents and risk groups in immunization programs. The first vaccine generation was based on HBsAg purified and inactivated obtained from plasma of asymptomatic carriers. These preparations were immunogenic; nevertheless, they were substituted quickly by recombinant vaccines produced in yeasts (Zuckerman JN. J MED VIROL 2006; 78: 169-177), having higher security. At present, research is focused on the development of formulations more immunogenic and effective, starting from the introduction of potent adjuvants, but these formulations have not been successful for the treatment of the chronic infection. Since the decade of the 90's of the past century, research to evaluate the use of the vaccination as treatment of the chronic infection by the HBV is conducted. This therapeutic strategy has received a remarked interest, coming from the results evidencing the fundamental role of the immune response in the control of this virus. Research teams working on this topic look for subverting the immune tolerance state to the HBV, starting by the administration of vaccine formulations (Hui CK, et al. INT J MED SCI. 2005; 2: 24-29).

Recent results in the field of the immune therapy, using the conventional preventive vaccines with therapeutic purposes, suggest the necessity to investigate the use of more potent vaccine candidates, that include new antigens, adjuvants, new administration routes and rational combined therapies (Pol S, et al. Expert Rev Vaccines 2006; 5: 707-16). The design of optimum vaccine candidates should keep in mind the aspects related with the antigenic component, as well as those related with the adjuvant component of the formulation.

The Center for Genetic Engineering and Biotechnology (CIGB) produce the recombinant HBsAg, using the yeast *Pichia pastoris* as host cell. This antigen is part of the Cuban anti-hepatitis B vaccine approved and used from the beginnings of the 90's (Muzio V, et al. European patent No. EP 480525).

The HBsAg has the capacity to induce a strong specific immune response, of antibodies and helper T cells in healthy people. Additionally, it has been demonstrated that HBsAg epitopes can be presented by molecules of the class I major histocompatibility system (HLA), even when this protein is processed by the antigen presenting cells like an exogenous protein. This property allows the non adjuvated HBsAg to induce a cytotoxic response by epitopes generated by alternative processing routes, allowing the expansion of the repertoire of HBsAg-specific cytotoxic cells (Bertoletti A, J Hepatol 2003; 39: 115-124). However, the use of this antigen for the therapeutic immunization has not resulted in reproducible benefits in Phase II studies. At present, a therapeutic vaccine against the chronic infection for the HBV does not exist.

One of the strategies used to favor the immune response in chronic patients is the use of the HBcAg as vaccine antigen. A remarkable characteristic of the amino acid sequence of the HBcAg is the presence, in the terminal carboxyl end, of a region rich in arginine. This region has the function of joining the pre-genomic ribonucleic acid (RNA) in the natural particle. In the case of the recombinant particle, this region is associated with bacterial RNA, with sizes in a range from 30 to 3000 nucleotides, which confers important immunologic properties (Riedl P. J Immunol 2002; 168: 4951-59).

The concept "immunotherapy" in the treatment of the HBC implies the reactivation of a defective immune response with the objective of controlling the viral replication and eliminating or controlling the infection. This concept has been validated directly in studies of bone marrow transplant. It could be demonstrated that in bone marrow transplant of cells from donors with immunity to the HBV to receptors chronically infected by this virus, this procedure was able to cure the chronic infection of the receptor patients (Lau GK, Gastroenterology 2002; 122: 614-24; SHOUVAL D, ILAN Y. J HEPATOL 1995; 23: 98-101). However, the immunization of non-immune donors to the HBV with the commercial vaccine did not produce a therapeutic response in patient receptors of bone marrow lymphocytes, producing an important limitation to that industrial applicability of this therapeutic strategy, due to its dependence of the natural infection of the donors.

During the chronic infection by the HBV the response of T and B effectors cells is poor, absent or functionally deficient in effector cells and professional antigen presenting cells. However, the patients that develop self-limited or acute infections show a strong cytotoxic and helper response, polyclonal and multi-specific against the proteins of the surface, the nucleocapsid and the polymerase (Webster GJ, J Virol 2004; 78: 5707-19). On the other hand, during the chronic infection the levels of Th1- associated cytokine secretion by lymphocytes of peripheral blood stimulated with the different viral antigens is very poor, on the contrary of what happens during the acute infection (Jung MC. Virology 1999; 261: 165-72; PENNA A, HEPATOLOGY 1997; 25: 1022-27). These discoveries suggest that potentiate the specific immune response can constitute a therapeutic alternative.

Another interesting discovery is that the patients that recover spontaneously of the HBC, or those that respond to the treatment with IFN-α, are able to develop cytotoxic responses of intensity similar to the one in patients that develop the acute infection

(Rehermann B, J Clin Invest 1996; 97: 1655-65). Additionally, an increase in the levels of CD4+ and CD8+ cellular response in patients treated with Lamivudine was demonstrated, suggesting that it is possible to induce an immune response starting from the sustained suppression of the virus (Boni C, Hepatology 2001; 33: 963-71; BONI C, J HEPATOL 2003; 39: 595-605).

Among immunotherapeutic strategies, the therapeutic vaccination constitutes an alternative explored during the decade of 1990 in the treatment of patients chronically infected by the HBV. The main immunotherapeutic strategies have focused in the use of the surface and viral nucleocapsid antigens.

The importance of the cellular immunity against the HBcAg in the control of the chronic infection by the hepatitis B was demonstrated by means of adoptive transfer. The studies carried out in patient with CHB, subjected to adoptive transfer of bone marrow cells of immune donors, evidenced that it is possible the elimination of the HBsAg of the serum of these patients. The study of the patients that were able to eliminate the HBsAg with this treatment allowed to evidence that the elimination of the chronic infection by the HBV was related to the transfer of CD4+ T lymphocytes specific for the HBcAg (Chee-Kin H, Int J Med Sci 2005; 2: 24-29).

### Immunotherapy with antigen presenting cells

The dendritic cells are professional antigen presenting cells of the immune system that instruct and control the activation of T and B lymphocytes, that are mediators of the specific immunity. From 1996 up to 2004, more than 60 clinical studies applying dendritic cells loaded or pulsed with tumoral antigens were carried out (Steinman RM. Nature 2007; 449: 419-426). Most of these studies have been carried out in melanoma patients. In many patients the clinical response matches with the induction of specific cytotoxic T response (Figdor CG. Nat Med 2004; 10: 475-480). Apparently, the clinical evolution of patients with cancer treated with dendritic cells is not satisfactory.

At present, the experts in the field of immunotherapy using dendritic cells (the most advanced among all the presenting cells used in immunotherapy) predict that there is an strong possibility that antigen-pulsed dendritic cells be more effective in patients that are not affected by cancer, due to the reduced damage in their immune system (Onji M, Akbar SMF. Dendritic cells in clinics. 2008; 2nd edition, Springer). However, this still remains as a chimera.

### Description of the invention

The present invention solves the previously mentioned problem, providing an optimum formulation for the stimulation of homologous or heterologous cells. The formulation object of the present invention is formed by particles of the HBsAg and HBcAg antigens in suspension. In addition, a method of cellular stimulation with this formulation, of specific and appropriate proportions, is disclosed, as well as the further passive immunization of CHB patients, based on the maximum stimulation, *in vivo* or *in vitro,* of heterologous or autologous cells that include dendritic cells, B cells and macrophages. The formulation of the invention contains the HBsAg and HBcAg antigens as mixtures of particles in suspension, of sizes lower than 500nm and higher than 500nm, in a proportion of particles of these sizes that goes from 50% - 50% to 80% - 20%, respectively.

It is also object of the present invention, a method of passive immunization characterized by the fact that the cellular stimulation is carried out by the *in vivo* immunization of uninfected persons -donors - with the formulation that contains the HBsAg and HBcAg antigens, the extraction of the immunocytes specifically activated, and its further passive transfer to patients chronically infected with the HBV. In an alternative embodiment of the invention, the cellular stimulation is described by the *in vivo* immunization of uninfected persons -donors -, the extraction of the specifically activated immunocytes, its *in vitro* re-stimulation with the formulation of the HBsAg and HBcAg antigens, and its furhter passive transfer to patients chronically infected with the HBV. In another embodiment of the invention, the cellular stimulation takes place by the extraction of immunocytes of persons non-immune to the HBsAg or the HBcAg, its *in vitro* stimulation with the formulation of the HBsAg and HBcAg antigens, and its further passive transfer to patient. In another embodiment of the invention, the cellular stimulation is carried out by means of the extraction of patient's immunocytes, its *in vitro* stimulation with the formulation of the HBsAg and HBcAg antigens, and the further passive transfer to the same patient.

In the present invention, for the antigenic stimulation, HBsAg and HBcAg precipitates has been obtained, as well as formulations of these antigens that are, for their physical-chemistry nature, optimal for their use as stimulating elements. The HBcAg has been produced as a nucleoprotein that contains small, but significant, quantities of Toll-like Receptors (TLR) ligands, as the deoxyribonucleic acid (DNA) and the RNA.

In the invention, these antigens have been precipitated by a method that allows the physical association of the HBsAg and of the HBcAg. This method makes that the levels of stimulation of the cells are maximum both in transgenic mice as in humans, inducing a reduction in the levels of HBsAg and viral load, respectively. In this way, the functionality and importance of this type of passive immunization in the treatment of the chronic infection by HBV was demonstrated. In this invention, the passive immunization has been evidenced with peripheral blood mononuclear cells, B cells, dendritic cells and macrophages, which have demonstrated its capacity to control the levels of the HBsAg and of the viral load in patients.

The present invention offers a solution to the problem that exists in the state of the art by means of potentiating the anti-HBsAg and anti-HBcAg immune response in donors, starting from the active immunization of the donors with a formulation that contains the HBsAg and HBcAg antigens and, in addition, the *in vitro* activation of the cells to be transferred, previous to the transfer, so that the response against these antigens is maximum when the cells are inoculated in the receptor organism. The results obtained as a result of the implementation of the present invention demonstrate the importance of obtaining the antigens with an optimum capacity of assimilation, activation and maturation of APC, and of the methods that are employed to obtain effective immune responses.

It is also an object of the present invention, the use of a formulation composed by particles of the surface antigens (HBsAg) and of the nucleocapsid (HBcAg) of the virus of the Hepatitis B (HBV) in suspension for the cellular stimulation and the active immunization, simultaneously or not, of patient with HBC.

### Brief description of the figures

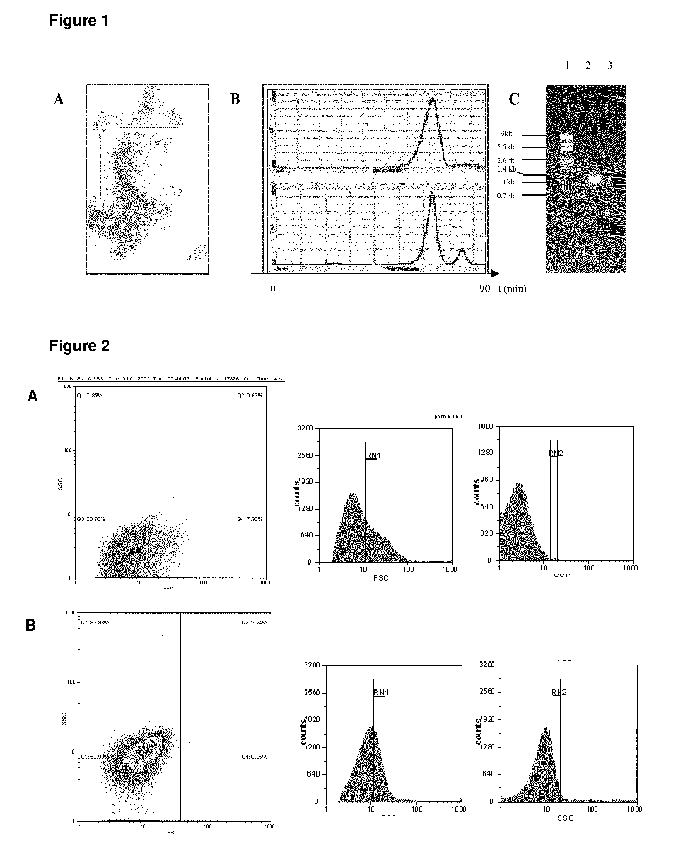

**Figure 1****.** Study of the physical aspect, chromatographic profile and presence of nucleic acids associated to the HBcAg. **A**. Transmission electron microscopy, the bars size is 200nm. **B**. Study of the size and homogeneity of the particle by high performance liquid chromatography (HPLC): the profile of the HBsAg (upper panel) and HBcAg (lower panel) are shown. Runs were carried out in columns TSK-G6000, at a flow of 0.25 mL/min in PBS, 100 µg of each protein were applied. The detection was carried out at 280 nm. Both particles had a retention time of 73 minutes. The second peak in the lower chromatogram corresponds to a non protein component of the solution in which the HBcAg is diluted (ethylendiamine tetra-acetic acid). **C**. Detection of the presence of nucleic acid associated to the HBcAg in agarose electrophoresis. Lane 1. DNA Molecular weight pattern, 2. HBcAg, 3. HBcAg treated with RNAse (2 mg/mL) at 37 °C during 6 h. The concentration of the HBcAg treated was of 0.447 mg/mL.

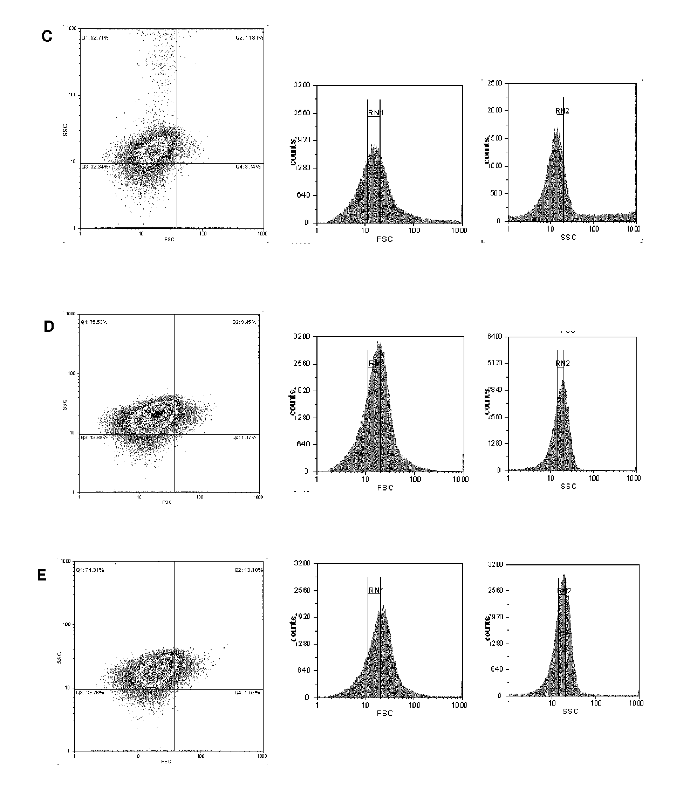

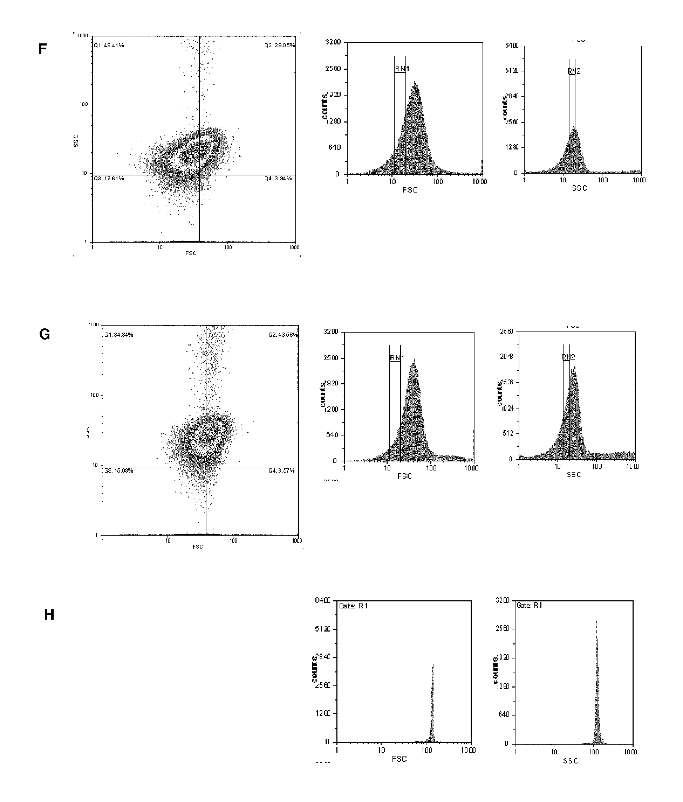

**Figure 2****.** Study of the increment in size of the complex HBsAg - HBcAg in solution by means of flow cytometry. **A.** Formulation of the HBsAg and HBcAg antigens in 1X PBS pH 7.0. **B.** Formulation after 1 h of dialysis against a solution of 50 mm Acetic Acid at pH 4.0. **C.** Formulation after 2 h of dialysis against a solution of 50 mm Acetic Acid at pH 4.0. **D.** Formulation after 3 h of dialysis against a solution of Acid Acetic 50 mm at pH 4.0. **E.** Formulation after 5 h of dialysis against a solution of 50 mm Acetic Acid at pH 4.0. **F.** Formulation after 8 h of dialysis against a solution of 50 mm Acetic Acid at pH 4.0. **G.** Formulation after 24 h of dialysis against a solution of 50 mm Acetic Acid at pH 4.0. **H.** Flow Cytometer calibration pearls size 2.5 micrometers.

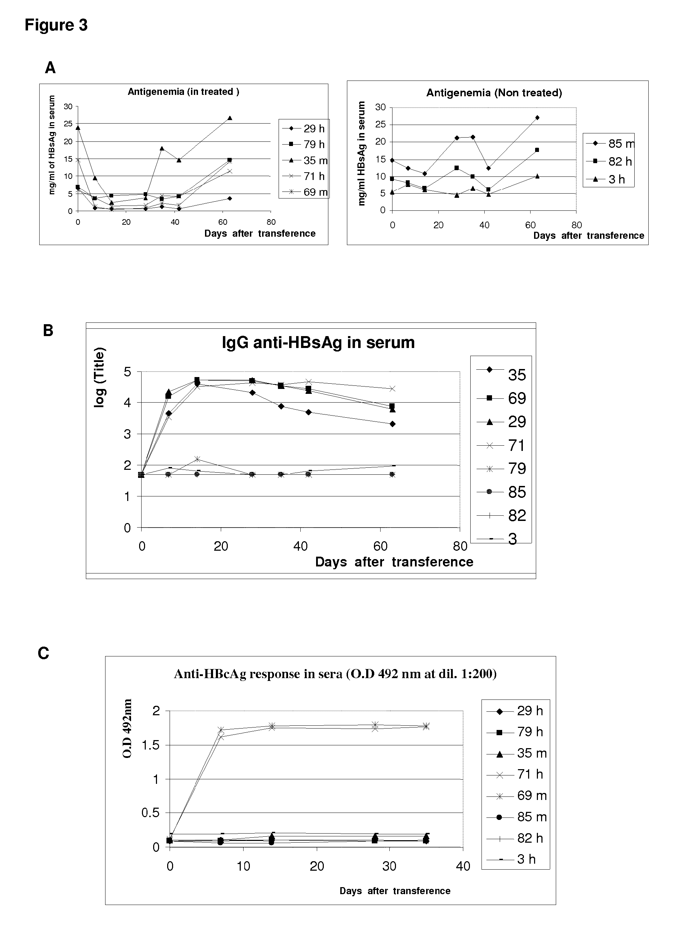

**Figure 3****.** Studies of passive immunization in transgenic mice starting from donors immunized with a formulation of HBsAg and HBcAg antigens. **A.** Dynamics of the antigenemia (concentration of HBsAg in µg/ml) in the serum of animals treated and non treated, at the different evaluation times. **B.** Anti-HBsAg IgG antibodiy response in the serum of the mice. **C.** Anti-HBcAg IgG antibody response in the serum measured by ELISA.

### Detailed description of the invention/ Examples

### Example 1. Generation of acid precipitates of the surface and nucleocapsid antigens of the HBV in suspension.

The HBcAg (CIGB, Cuba) was expressed as a 183 amino acids recombinant protein purified from *Escherichia coli.* The recombinant HBsAg, subtype adw2, was produced in *Pichia pastoris* (CIGB, Cuba). The fermentation and purification process of the HBcAg protein was carried out as previously described (Aguilar JC. Immunol Cell Biology 2004; 82: 539-546). The purification process of this antigen results in a highly purified nucleocapsid antigen (>95%) as it can be seen in Figure 1A. This antigen has a similar molecular size to the HBsAg, it means that also forms virus-like particles (Figures 1B), and additionally contains traces of nucleic acids associated to its chemical structure, which function as TLR stimulators as it can be seen in Figure 1C. These ligands of TLR associated in traces can be characterized, mainly, as RNA and a residuary quantity of DNA, both of bacterial origin.

The presence of nuclear material associated to the purified HBcAg protein could be detected by an increment in the absorbance at 260 nm, with regard to other proteins such as the HBsAg and bovine serum albumin, which have half of the absorbance at 260 nm when they are in equal concentration than the HBcAg (data not shown). However, to study if the nuclear material was associated to the particle, an agarose gel electrophoresis was performed, in presence of ethidium bromide. This experiment demonstrated the presence of nuclear material in the same zone where the protein was detected.

The treatment with the RNAse enzyme allowed to eliminate most of the nuclear material associated to the particle, evidencing that the RNA constitutes the higher percentage of the nuclear component of this nucleoprotein (Figures 1C, lanes 2 and 3). In synthesis, these results show the physical aspect of virus-like particle, and the nucleoprotein nature of the recombinant HBcAg protein, and that the main part of the nucleic acid is RNA, even when there is also DNA associated to it.

For the generation of HBsAg and HBcAg precipitate suspensions a dialysis of the mixture of both antigens against a solution of 50mM Acetic Acid pH 4.0, at protein concentrations between 0.1 and 2 mg/mL was carried out. The precipitate suspension was selected according to the particle size, limiting the time of dialysis to periods between 10 min and 24 hours. An alternative method was the change of the buffer solution of the HBsAg - HBcAg mixture. This could be carry out by diafiltration, using fiber cartridges with pores of sizes inferior to the size of the antigens (<20 nm). In this case the acid precipitation process takes place during the adjustment of pH value to 4.0. In both precipitation methods the exposure to the pH changes defines the size of the precipitate. The precipitate is selected according to its size and depending on that feature are the cellular types stimulated after immunization with these preparations.

Later, the precipitates are adjuvated or not, depending on the formulation that is required. One variant constitutes the absorption to aluminum hydroxide, but absorption to other adjuvants of the group of depot effect adjuvants (salts and oily adjuvants) or immunostimulants (lipopolisaccharides, saponins and its derivatives) has been done.

### Example 2. Characterization of the molecular size of the HBsAg and HBcAg antigen suspensions, and process for the selection of the optimum sizes for the assimilation and antigen stimulation.

With the objective of selecting the precipitate of optimal sizes for the stimulation of professional antigen presenting cells, four precipitation variants were selected. The precipitation variants were produced by the method described in the Example 1, both for dialysis variants and for diafiltration and pH adjustment at 4.0, with predetermined incubation times.

The selection of the size was carried out by flow cytometry, using for calibration a size pattern pearls of 2.5 micrometers as higher limit. A range between 50 nm and 2.5 micrometers was employed in the experimentation, evidencing that, in a general, the assimilation and cellular stimulation differs in dependence of the size of the precipitate and of the cell type. The macrophages seem to be the cells that assimilate more easily the precipitate from sizes higher than 500 nm. In this way, precipitate mixtures were selected to increase the power of stimulation of the antigenic mixture. Consequently, higher levels of stimulation and immunogenicity were obtained with these mixtures compare to those obtained with the non-precipitate antigens.

Figure 2 shows the graphics and histograms resulting from the application of different precipitate to the flow cytometer. A shift in the "Forward Scatter" level was evidenced, representing an increment in the size of the obtained particles. This size increment occurs in direct proportion with the incubation time of the mixture of antigens (HBsAg and HBcAg). An increment in the "Side Scatter" levels was also observed, which is a reflection from the inherent rugosity of the particle in suspension, formed by antigens of 22 nm in sizes that achieved the 2.5 micrometers, as it is observed if the bead pattern of 2.5 micrometers is taken into account, as it is shown in Figure 2H.

Considering that the flow cytometry allows establishing a system of quadrants and ranges in histograms, the selection of the precipitated particles was carried out in function of its coordinates in the quadrants with established ranges or by the establishment of ranges in the histogram graphics. In Figure 2, the results of incubations of the antigenic mixture in the dialysis are represented, allowing the selection of precipitate of different sizes. Similar results were obtained by direct precipitation, reducing the pH of the acetate buffer solution from pH 7.0 to pH 4.0; but in different ranges of time. The final selection was carried out by flow cytometry and **immunogenicity criteria.**

### Example 3. Studies of passive immunization using adoptive transfer of cells from Balb/c mice, previously immunized with formulations comprising the HBsAg and HBcAg antigens, to transgenic Balb/c mice expressing HBsAg.

The immunotherapy, based on the development of vaccine candidates, constitutes one of the strategies in the chronic Hepatitis B therapy. The effectiveness of the candidates depends on the generation of a potent humoral and cellular immune response able to overcome the state of tolerance to the viral antigens that characterizes this illness.

In the present invention the anti-HBsAg and anti-HBcAg immune response in donors is potentiated by active immunization with a formulation that contains the HBsAg and the HBcAg. Additionally, the cells to be transferred are activated *in vitro,* prior to the transfer, so that the response against these antigens is optimum when the cells are inoculated in the receiving organism. By this artificial way, a type of response non existent in humans was generated, allowing to increase the margins of donor including people with similar haplotypes, with independence of if they have been infected by HBV or not.

In the present example, it is evaluated by means of the adoptive transfer of cells, the effect of the immune response, generated by the vaccination with a vaccine candidate containing HBsAg and HBcAg antigens applied by the nasal/parenteral routes, in the context of a transgenic mouse that express the HBsAg as a model of HBV persistent infection. One of the objectives of the study was the evaluation of the effect of the transferred immune response on the concentration of HBsAg (antigenemia) in the serum of the transgenic mouse. Also, the effect of the response induced by the active immunization by nasal/parenteral routes with the described formulation was compared to the effect generated by the adoptive transfer of cells stimulated with the HBsAg *in vivo* and *in vitro.* In addition, the kinetics of the transferred anti-HBsAg antibody response was studied in the context of the transgenic mouse for this antigen. The effect of the adoptive transfer of immunity on the hemochemistry and histological parameters was evaluated, as a measure of safety of the vaccine candidate applied by nasal/parenteral routes. In this study, Balb/c mice (H-2d haplotype, coming from CENPALAB, Cuba) and HBsAg (+) transgenic mice (with a Balb/c genetic bottom, obtained at the CIGB) were used.

### Generation of anti - HBsAg immunity in Balb/c mice.

An immunization schedule was carried out in Balb/c mice, female, between 8 to 12 weeks of life. Mice were immunized with the vaccine candidate that contains HBsAg and HBcAg antigens, simultaneously by the intranasal (IN) and parenteral routes, in this last case the intramuscular (IM), subcutaneous (SC) and intradermal (ID) routes were evaluated. The doses (in volume of 100 µL) were administered on days 0 and 14, and a booster dose was administered on day 100, previous to the transfer. The blood extractions were carried out by the retrorbital plexus on days -2, 10 and 25. Table 1 reflects the immunization schedule design.

**Table 1. Immunization schedule in non-transgenic Balb/c mice.**

| Group | Treatment | Route | Number of animals |
|---|---|---|---|
| 1 | HBsAg + HBcAg + alumina / HBsAg + HBcAg | IM / IN | 13 |
| 2 | HBsAg + HBcAg + alumina / HBsAg + HBcAg | SC / IN | 13 |
| 3 | HBsAg + HBcAg + alumina / HBsAg + HBcAg | ID / IN | 13 |
| 4 | HBsAg + HBcAg / HBsAg + HBcAg | IM / IN | 13 |
| 5 | HBsAg + HBcAg / HBsAg + HBcAg | SC / IN | 13 |
| 6 | HBsAg + HBcAg / HBsAg + HBcAg | ID / IN | 13 |
| 7 | alumina / PBS | IM / IN | 9 |

The evaluation of the humoral immune response generated by these treatments was carried out by ELISA. The anti-HBsAg IgG and the IgG subclases after each dose were measured. The cellular immune response was evaluated, by ELISPOT technique, 10 days after the first administration. The ELISPOT employed was designed to measure the anti-HBsAg specific gamma-IFN secretion by CD8+ splenocytes. The results of these evaluations indicate that the immunization group 5 generates the highest cellular response and a humoral response similar to the rest of the groups studied. Based on this, two animals of this group were selected as splenocyte donors for the adoptive transfer. The selected immunization dose was a 1:1 (HBsAg:HBcAg) proportion, with a quantity of antigen precipitated in suspension in a range between 10% and 50%. Other proportions have also been evaluated.

### Obtention of immune splenocytes.

Fifteen days after receiving the booster dose two mice of the group 5, and three mice of the group 7 (placebo) were sacrificed and the spleens were extracted. The spleens of the group 5 and those of the group 7 were grouped, respectively. The spleens were processed for splenocytes purification as usually. The splenocytes were separated in aliquots of 30 x 10⁶ cells in 100 µL of phosphate saline buffer solution (1X PBS), for their transfer to the receptor mice. Two of these aliquots received a re-stimulation previous to the transfer, for that they were incubated with 500 µL of the formulation comprising HBsAg and HBcAg antigens, and 500 µL of RPMI supplemented media each. The incubation was carried out in 15 ml tubes during 1 h at 37ºC and 5% of CO₂. The samples were agitated each 15 min. After incubation, these two cell aliquots were washed and suspended separately in 100 µL of 1 X PBS for the transfer.

### Adoptive transfer of immunity.

Transgenic mice that express HBsAg were used as receptors. The transgenic mice employed were of both sex and between 16 to 20 weeks of life. They were assigned to different treatment groups as shown in Table 2. Previous to the splenocyte transfer, a partial blood extraction was carried out to check the HBsAg basal levels in serum. Two days after, 30 x10⁶ splenocytes, in a volume of 100 µL of 1X PBS, were intraperitoneally (IP) administered. Blood extractions, to evaluate the effect of the adoptive transfer of immunity, were carried out weekly during 5 weeks through the retro-orbital plexus. On week 8 post-transfer, the animals were measured and weighted, and after that they were bled and sacrificed. The main organs were weighed and preserved in formaldehyde for histological analysis. Part of the blood extracted in this experimental point was used in the determination of hemochemistry parameters such as hepatic transaminases (ALT), alkaline phosphatase and creatinine.

**Table 2. Design of the experiment of adoptive transfer of immunity.**

| Group | Treatment | Number of animals | Identification of the mice |
|---|---|---|---|
| 01 | Transfer (IP) of splenocytes of Balb/c mice with HBsAg and HBcAg response | 3 | 29,35,79 |
| 02 | Transfer (IP) of splenocytes of Balb/c mice with HBsAg and HBcAg response and previous *in vitro* re-stimulation | 2 | 69, 71 |
| 03 | Transfer (IP) of splenocytes of placebo Balb/c mice | 2 | 3, 67 |
| 04 | PBS 1x (IP) | 2 | 82,85 |

### Quantification of HBsAg in the serum of HBsAg transgenic mice.

The ELISA protocol used to determine the levels of HBsAg in serum was developed in our own laboratory. ELISA plates were coated during 20 min at 50ºC with the anti-HBsAg monoclonal antibody named Hep4 (CIGB, Cuba). Later on, a series of washes with PBS 1X-0.05% Tween 20 were carried out, and the plates were incubated during 1 h at 50ºC with the serum samples, diluted 1/200 in 1 X PBS with 2% skim milk. In this step, an HBsAg (CIGB, Cuba) standard curve of known concentration was introduced in each plate. The standard was serially diluted starting at the concentration of 141 ng/ml.

As a negative control a mixture of pre-immune sera of non transgenic Balb/c mice was used. Once elapsed the incubation time the washes were carried out, and the plate was incubated for 30 min at 50ºC with the anti-HBsAg monoclonal antibody Hep1 conjugated to peroxidase, diluted in buffer (0.44 g of skim milk, 220 µL Tween 20 in 11 ml of distilled water). After a series of washes the plate was incubated for 10 min at room temperature with the substrate (citrate-phosphate buffer; 1 mg/mL *orto*phenylendiamine; 0.1% H₂O₂). Finally, the reaction was stopped adding 50 µL/well of 3M H₂SO₄. The resulting optical density (O.D) was read at 492 nm using a plate reader (Sensident Scan, Merck).

All mice that received cells with previous HBsAg immunity showed a marked decrease of the HBsAg in serum since the one week post-transfer evaluation, finding significant differences among time zero and the second and third week (p<0.05). From the fourth week (day 35) on, it is observed that HBsAg concentration in serum began to increase, indicating that the control of the antigenemia in the transferred mice began to disappear. Since this time point and until week 8 (day 63) statistical differences in the antigenemia with respect to time zero were not observed (Figures 3A).

As it is shown in Figure 3A, in the case of mice that received splenocytes with HBsAg specific immunity a marked decrease of the HBsAg concentration in serum was detected, which was more remarkable between day 7 and 28. However, for mice that received the placebo splenocytes or were immunized with saline solution, although fluctuations are detected in the HBsAg concentration in serum, the values never significantly differed from the time zero, and never values below 5 µg/ml were detected.

These results indicate that it is possible, through the adoptive transfer of cellular immunity, to efficiently diminish the levels of circulating HBsAg in the serum of the transgenic animal. The control established on the antigenemia as a result of the transferred immune response was effective, lasting around three weeks after a unique cell transfer.

Another experiment carried out later evidenced that this effect is associated to the stimulation of the HBcAg on the HBsAg, considering that the donor animals stimulated with a formulation that only contains the HBsAg did not produce any control over the antigenic load, and generated lower responses in these mice compared to those evidenced with the HBsAg and HBcAg combined formulation.

### Anti-HBsAg IgG response in sera.

An HBsAg- specific IgG antibody response was detected for all mice that received splenocytes with previous immunity to this antigen, with the exception of mouse 79 (Figures 3B). This agrees with the antigenemia results obtained for this specific animal, in which there was only a slightly decrease of the HBsAg concentration. In the animals with a positive anti-HBsAg response the titers detected were high (> 10⁴) and began to diminish from the third week on. This could be related with the increment of the antigenemia observed for these animals around the fourth week (day 35). The groups that received transfer of placebo cells or saline solution did not show HBs-specific antibodies titers.

### Evaluation of the hemochemical parameters.

At the end of the study, on week 8, several hemochemical parameters were evaluated. The hemochemical study was designed with the objective of measuring the potential organic damage, due to adoptive transfer of HBsAg-immune splenocytes to transgenic mice that constitutively express the HBsAg in liver, kidneys and other organs. Determinations of ALT and alkaline phosphatase, both markers of the hepatic function, and creatinine as a marker of the renal function, were carried out. The values obtained for ALT and creatinine in all mice (treated and placebo) were in the range of normal values for these animals.

The statistical comparison between treated (that received splenocytes with anti-HBsAg immunity) and no treated (that received placebo splenocytes or 1X PBS solution) did not show significant differences for any of the evaluated parameters. The comparison carried out per sex showed differences only in the case of the alkaline phosphatase, obtaining higher values for males.

### Histological analysis.

On week 8 (day 63) post - transfer, all the animals of the study were sacrificed and all relevant organs extracted and weighed. The data of the animals including age at the beginning of the treatment and weight and size at the end, revealed that none significant differences exist as a result of the treatment. The extracted organs were included in formaldehyde for histological analysis. Some statistical comparisons among the measured parameters were carried out; the results indicate that do not exist differences with respect to the animal neither to the organs weight between treated and no treated animals. Some differences between sexes were found, and higher values for some organs in the case of males were obtained.

### In vitro re-stimulation enhance the serum anti-HBcAg immune response in mice that received the adoptive transfer of immune splenocytes.

Considering that the immune splenocytes transferred to the Balb/c transgenic mice came from mice with a potent anti-HBs and -HBc immune response, we decided to evaluate if the anti-HBc response was detectable in the serum of the receptor mice.

As shown in the Figure 3C, only those mice that were transferred and received previous *in vitro* re-stimulation with the combined formulation (mice 69 and 71) conserved a high IgG antibody response against the HBcAg.

The animals that received splenocytes with previous anti-HBcAg immunity but did not were re-stimulated *in vitro* (mice 29, 35 and 79) did not show HBcAg-specific IgG response in serum, similar to the one observed in mice transferred with placebo splenocytes or immunized with 1 X PBS. This result evidences that is possible potentiate the anti-HBcAg immune response when this type of immunization was carried out.

Although in this study a specific variant of precipitate mixture was selected with different sizes (50% of the precipitate had sizes smaller than 500nm and the other 50% had sizes higher than 500nm), the proportions of precipitated in suspension of the HBsAg and the HBcAg used had a differential effectiveness, the results of the evaluations of mixtures of precipitates of different sizes allowed to direct the immunogen to different antigen presenting cell populations, this have an impact in the assimilation, capacity of cellular activation and immunogenicity of the final preparation.

The results obtained in the antigen presenting cell activation were surprisingly superior when the formulations of the antigens were used in suspension with respect to the same formulation without treatment.

Taking all together the results obtained before, it can be concluded that the adoptive transfer of immunity was effective, because it diminished the levels of circulating HBsAg in the serum, something that was not reach with the active immunization using HBsAg and HBcAg combined formulations. Also, the adoptive transfer of immunity described previously resulted safe, since histological damages were not appreciated in the studied organs. These results demonstrated the safety of the immunotherapy, because is transferred an immunity able to suppress for more than two weeks, and even in some cases became undetectable, the serum HBsAg concentration in the evaluated model. In addition, it was proved the benefit of the *in vitro* re-stimulation with the HBsAg and HBcAg previous to the transfer, favoring the presence of an anti-HBcAg immune response, which is of vital importance in humans.

### Example 4. Transfer of heterologous cells of non immunized mice, pulsed in vitro with formulations of the HBsAg and HBcAg antigens.

With the objective of demonstrate that heterologous cells stimulated or pulsed *in vitro* with mixtures of the HBsAg and HBcAg antigens induce a strong immune response against these antigens, an experiment in Balb/c mice, transgenic to the HBsAg and non transgenic was carried out. The non transgenic Balb/c mice were employed as donors.

Eight Balb/c mice were sacrificed and their spleens were extracted. The spleen cells were purified according to the methodology explained in the Example 3. The total spleen cells, or specific cellular populations (B cells, dendritic cells and macrophages) obtained from these spleen cells, were *in vitro* stimulated with different formulations and proportions of the antigens, and at different supernatant concentrations. After the stimulation, the cells were washed three times and transferred to HBsAg transgenic and non transgenic mice.

All the transferred Balb/c mice, transgenic and non transgenic, generated an immune response detectable. This response was able to induce a significant reduction of the HBsAg levels in those receptor mice belonging to the HBsAg transgenic group that it was detectable for 1 to 20 weeks, and in some cases in a permanent way. This result was not reached by the active immunization using the same formulations (data not shown).

### Example 5. Transfer of homologous cells of non immunized mice, pulsed in vitro with formulations of the HBsAg and HBcAg antigens.

With the objective of demonstrating that stimulated homologous or cells *in vitro* pulsed with mixtures of the HBsAg and HBcAg antigens, induce a strong response against these antigens, an experiment in Balb/c, HBsAg transgenic and non transgenic mice was carried out. The non transgenic Balb/c mice were employed as splenocyte donors.

Eight Balb/c mice were sacrificed and the spleens were extracted. The spleen cells were purified according to the methodology explained in the Example 3. The total spleen cells or the specific spleen cell populations (B cells, dendritic cells and macrophages) were *in vitro* stimulated with different formulations and proportions of the antigens, and at different supernatant concentrations. After the stimulation, the cells were washed three times and transferred to HBsAg transgenic and non transgenic mice.

All the transferred Balb/c mice, transgenic and non transgenic, generated a detectable immune response. This response was able to induce a significant reduction of the HBsAg levels in those receptors mice belonging to the HBsAg transgenic group. The reduction of the HBsAg concentration in sera was detectable for 1 to 20 weeks, and in some cases in a permanent way. This result was not reached by the active immunization using the same formulations (data not shown).

### Example 6. Study of the antigenic stimulation of dendritic cells using different antigens and antigenic mixtures of different particle sizes and proportions.

An experiment carried out in transgenic mice evidenced that the stimulation effect of the antigens on the cells is related to the stimulation generate by the HBcAg on the HBsAg, and to the nature of the interaction between the HBcAg and the HBsAg, which is associated with the particles size and the proportions of different particles sizes in the mixtures.

If the animals used as donors were stimulated with a formulation containing only the HBsAg the control on the antigenic load is not produced. These animals generated lower responses with respect to animals stimulated with the HBsAg and HBcAg combined formulation. The mixtures of precipitate of different sizes produced the most potent stimulations in the different antigen presenting cell types. This result came from the measurement of the capacity to control the circulating HBsAg in transgenic mice.

In summary, the higher stimulation potency variant consists of a proportion that is in a range of 50 to 80% from sizes less than 500nm, and 20 to 50% from sizes of more than 500 nm. This range was studied stimulating antigen presenting cells mixtures that contain B cells, macrophages and dendritic cells, and transferring these cellular mixtures to the HBsAg transgenic mice model, in which the difference was evidenced in the capacity to control the HBsAg (Table 3). As, it can be appreciated, the variants E and F shows the optimum range of stimulation.

**Table 3. Serum HBsAg concentrations in transgenic mice transferred with cells stimulated with different treatments.**

| Stimulation Treatments | HBsAg Concentration (µg/mL) | |
|---|---|---|
| | Time 0 days | Time 21 days |
| A- HBsAg en PBS | 18.5 +/- 2.3 | 17.5 +/- 3.3 |
| B- HBsAg/HBcAg en PBS | 16.5 +/- 1.7 | 11.8 +/- 2.1 (*) |
| C- 100% HBsAg/HBcAg (<500nm) | 16.2 +/- 1.9 | 5.5 +/- 1.7 (**) |
| D- 100% HBsAg/HBcAg (>500nm) | 16.9 +/- 2.9 | 4.5 +/- 1.4 (**) |
| E- 50% (C) + 50% (D) | 16.2 +/- 3.0 | 1.0 +/- 1.7 (***) |
| F- 80% (C) + 20% (D) | 16.3 +/- 3.5 | 0.1 +/- 1.7 (***) |
| G- 20% (C) + 80% (D) | 16.2 +/- 3.0 | 5.1 +/- 1.6 (**) |

The stimulation capacity of each treatment in macrophages, dendritic cells and B cells were characterized studying the expression of activation markers in the cell surface. The stimulation capacity was higher for the treatment mixtures E and F. This result can be in line with the higher antigen assimilation capacity of each cell population involved in the mixture.

It is worth to be highlighted that the formulation B comprising the HBsAg and HBcAg mixture in solution (non precipitate formulation whose average particle size is lower than 50 nm), it is able to produce an immune response that, after been transferred to transgenic mice produces a significant decrease in the serum HBsAg concentration. However, the most important reductions in HBsAg concentration were produced by the C and D precipitate mixtures, showing in some cases sustained undetectable levels. The mixture G generated lower stimulation levels and HBsAg concentration decreases than mixtures E and F, evidencing the specificity of these formulations and the proportions established in the generation of potent immune responses by adoptive immunity transfer methods.

### Example 7. Passive immunotherapy studies in chronic Hepatitis B patients.

Patients with chronic HBV infection were separated for the study in four groups of 10 patients each. A first group was treated with peripheral blood mononuclear cells of haploidentical donors. The second group received cells purified from a previous auto-donation (homologous cells). In both cases the cells were stimulated with the HBsAg and HBcAg formulations. The other two groups received the same treatment but the re-inoculated cells were not stimulated with the antigens.

It could be evidenced that the patients transfused with stimulated cells developed an immune response, something that could not be appreciated in the patients receiving not stimulated cells. In line with that, significant HBsAg levels and viral load reductions were evidenced in the groups 1 and 2 with regard to the groups 3 and 4, and also compared to the values obtained before treatment.

### Example 8. Passive immunization studies in chronic Hepatitis B patients using pulsed macrophages, B cells and dendritic cells.

Patients with chronic HBV infection were separated in six groups, of 20 patients each for the study. A first group was treated with dendritic cells, isolated from peripheral blood monocytes of haploidentical donors. A second group received cells purified from a previous auto-donation (homologous cells).

Both groups were subdivided in two groups of 10 patients each that received either cells stimulated with the HBsAg and HBcAg formulations or not stimulated cells. The other two groups received the same treatments, but employing B cells. The two remaining groups were treated with adherent cells, enriched in macrophages.

The patients that were transfused with the stimulated cells generated an immune response, something that was not appreciated in the case of the patients that received non stimulated cells. In line with that, significant HBsAg levels and viral load reductions were evidenced in the groups that received the pulsed cells, evidencing the usefulness of the antigenic stimulation with the HBsAg and HBcAg antigens for the different cellular types.

This type of studies was optimized in the transgenic mice model, selecting the precipitated in suspension antigens populations in variable proportions or not. The obtaining of optimum stimulation variants allowed the increment of the resultant immune response.

## Claims

1. Formulation of the surface (HBsAg) and the nucleocapsid (HBcAg) antigens of the Hepatitis B virus (HBV) characterized because it comprises precipitates in suspension of said antigens.

2. The formulation of claim 1 wherein the precipitates in suspension constitute a mixture of particles of sizes lower than 500 nm and higher than 500 nm.

3. The formulation of claim 2 wherein the particles of sizes lower than 500 nm and higher than 500 nm are in a proportion that goes from 50% - 50% to 80% - 20%, respectively.

4. The formulation of claim 1 wherein the precipitates in suspension are obtained as a result of subjecting a mixture of these antigens to an acid precipitation.

5. The formulation of claim 4 wherein the acid precipitation is achieved by means of the dialysis or diafiltration against an acid solution.

6. Method to obtain a formulation of the surface (HBsAg) and the nucleocapsid (HBcAg) antigens of the Hepatitis B virus (HBV), formed by precipitates in suspension of these antigens characterized for: a) to prepare a mixture of the HBsAg and HBcAg antigens, b) to subject this mixture to an acid precipitation, and c) to select the precipitates according to their size, in the way that they produce the maximum *in vivo* or *in vitro* stimulation of heterologous or autologous antigens presenting cells.

7. The method of claim 6 wherein the concentration of the HBsAg and HBcAg antigens in the mixture that is subjected to acid precipitation is between 0.1 and 2 mg/mL of protein.

8. The method of claim 6 wherein the acid precipitation is achieved by means of dialysis or diafiltration against an acid solution.

9. The method of claim 8 wherein the acid solution for the dialysis or the diafiltration is Acetic Acid, preferably 50mM Acetic Acid at pH 4.0.

10. The method of claim 8 wherein the acid precipitation lapses for a period of time between 10 minutes and 24 hours.

11. The method of claim 6 wherein the size of the particles of precipitate is selected in the way that those particles with sizes lower to 500 nm and higher to 500 nm are in a proportion that goes from 50% - 50% to 80% - 20%, respectively.

12. Method of cell stimulation for the passive immunotherapy characterized because the stimulating agent is a formulation composed by precipitates in suspension of the HBsAg and HBcAg antigens of the Hepatitis B virus (HBV).

13. The method of claim 12 wherein the precipitates in suspension constitute a mixture of particles of sizes lower than 500 nm and higher than 500 nm.

14. The method of claim 12 wherein the population of cells to stimulate is coming from an individual selected from the group composed by: not infected individuals, individuals non immune to HBV, and patients of chronic Hepatitis B (CHB).

15. The method of claim 12 wherein the population of cells to stimulate is selected from the group composed by: peripheral blood mononuclear cells, dendritic cells, B cells and macrophages.

16. The method of claim 12 wherein the stimulation of the cells is produced by the *in vitro* stimulation of the cells isolated from an individual, the *in vivo* immunization of said individual, or both stimulation strategies are combined.

17. Method of passive immunization of CHB patients **characterized by**: a) to stimulate cells with a formulation composed by precipitates in suspension of the HBsAg and HBcAg antigens of the Hepatitis B virus (HBV) and b) to transfer the stimulated cells to a patient with CHB.

18. The method of claim 17 wherein the precipitates in suspension constitute a mixture of particles of sizes lower than 500 nm and higher than 500 nm.

19. The method of claim 17 wherein the population of cells to stimulate is coming from an individual selected from the group composed by: not infected individuals, individuals non immune to the HBV, and Chronic Hepatitis B patients (CHB).

20. The method of claim 17 wherein the population of cells to stimulate is selected from the group composed by: peripheral blood mononuclear cells, dendritic cells, B cells and macrophages.

21. The method of claim 17 wherein the stimulation of the cells is produced by the *in vitro* stimulation of the cells isolated from an individual, the *in vivo* immunization of said individual, or both stimulation strategies are combined.

22. The use of a formulation composed by precipitates in suspension of the surface (HBsAg) and the nucleocapsid (HBcAg) antigens of the Hepatitis B virus (HBV) for the cellular stimulation and the active immunization, simultaneously or not, of CHB patients.

23. The use of claim 22 wherein the precipitates in suspension constitute a mixture of particles of sizes lower than 500 nm and higher than 500 nm.

24. The use of claim 22 wherein the population of cells to stimulate is selected from the group composed by: peripheral blood mononuclear cells, dendritic cells, B cells and macrophages.

25. The use of claim 22 wherein the stimulation of the cells is produced by the *in vitro* stimulation of the cells isolated from an individual, the *in vivo* immunization of said individual, or both stimulation strategies are combined.

26. The use of claim 22 wherein the population of cells to stimulate is coming from an individual selected from the group composed by: not infected individuals, individuals non immune to the HBV, and Chronic Hepatitis B patients (CHB).
